(19) Europäisches Patentamt
European Patent Office
Office européen des brevets

(11) Veröffentlichungsnummer: **0 019 790**
**B1**

(12) # EUROPÄISCHE PATENTSCHRIFT

(45) Veröffentlichungstag der Patentschrift: **15.06.83**

(21) Anmeldenummer: **80102623.8**

(22) Anmeldetag: **12.05.80**

(51) Int. Cl.³: **A 61 K 49/02,** A 61 K 43/00, C 07 B 23/00, C 07 F 13/00, C 07 C 103/375, C 07 C 103/365, C 07 C 103/50

(54) **Technetium-99m-markierte Acetanilidoiminodiacetate zur Leberfunktionsdiagnostik und Verfahren zu ihrer Herstellung.**

(30) Priorität: **18.05.79 DE 2920174**

(43) Veröffentlichungstag der Anmeldung: **10.12.80 Patentblatt 80/25**

(45) Bekanntmachung des Hinweises auf die Patenterteilung: **15.06.83 Patentblatt 83/24**

(84) Benannte Vertragsstaaten: **AT BE CH DE FR GB IT LI LU NL SE**

(56) Entgegenhaltungen:
**FR-A-2 322 586**
**FR-A-2 353 299**
**JOURNAL OF NUCLEAR MEDICINE, Band 17, Nr. 7, Juli 1976, Seiten 633–638 New York, U. S. A. M. D. LOBERG et al.: »Development of newradiopharmaceuticals based on N-substitution of iminodiacetic acid«**

(73) Patentinhaber: **HOECHST AKTIENGESELLSCHAFT, Postfach 80 03 20, D-6230 Frankfurt/Main 80 (DE)**

(72) Erfinder: **Molter, Michael, Dr., Unter den Akazien 7, D-6000 Frankfurt am Main 70 (DE)**
Erfinder: **Kloss, Gerhard, Dr., Liederbachstrasse 14, D-6233 Kelkheim (Taunus) (DE)**

**EP 0 019 790 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Technetium-99m-markierte Acetanilidoiminodiacetate zur Leberfunktionsdiagnostik und Verfahren zu ihrer Herstellung

Die vorliegende Erfindung bezieht sich auf ein neues Präparat zur szintigraphischen Darstellung des hepato-biliären Systems (Leber, Gallenblase, intra- und extrahepatische Gallenwege). Das Präparat ist vor allem für dynamische Untersuchungen geeignet.

Für die nuklearmedizinische Diagnostik hat sich in den letzten Jahren Technetium-99m wegen seiner günstigen physikalischen Parameter (keine Korpuskularstrahlung, $\gamma$-Energie von 140 keV, Halbwertszeit von 6 h) und der damit verbundenen geringen Strahlenbelastung für Patienten und Personal, sowie wegen der einfachen Gewinnung mit Hilfe von Nuklidgeneratoren weitgehend durchgesetzt.

Das aus solchen Generatoren gewonnene Technetium-99m liegt zunächst als Pertechnetat vor und ist in dieser Form z. B. für die Schilddrüsen- und Hirnszintigraphie geeignet. Um auch andere Organe der Diagnostik mit Tc-99m zugänglich zu machen, wurden organspezifische Transportsubstanzen entwickelt, die gut mit Tc-99m markiert werden können und die so eine gute szintigraphische Darstellung der verschiedensten Organe zulassen. So kann man z. B. mit markierten Kolloiden die RES-haltigen Organe wie Leber und Milz darstellen; für die Knochenszintigraphie eignen sich bestimmte markierte Phosphorverbindungen und so weiter.

Für die Markierung der Transportsubstanzen mit Tc-99m wird im allgemeinen das sehr reaktionsträge Pertechnetat ($TcO_4^-$) zunächst in eine niedrigere Oxidationsstufe reduziert. In dieser Form ist Technetium dann reaktionsfreudig und bildet mit der entsprechenden Transportsubstanz einen relativ stabilen Komplex.

Die Reduktion des $TcO_4^-$ kann durch chemische Reduktionsmittel oder elektrolytisch durchgeführt werden; die Reduktion mit Zinn(II)-salzen ist heute am weitesten verbreitet.

Die Reduktion mit Zinn(II) hat den Vorteil, daß man das Reduktionsmittel und die organspezifische Transportsubstanz — im allgemeinen in gefriergetrockneter Form — in einer Injektionsflasche gemeinsam aufbewahren kann, so daß für die Herstellung des anwendungsfertigen Präparats in der Klinik lediglich das Generatoreluat zugegeben werden muß, welches das $^{99m}$Tc-Pertechnetat enthält.

Durch die zur Verfügung stehenden Detektoren war die nuklearmedizinische Diagnostik lange Zeit weitgehend auf die Lokalisation bestimmter Organe und die Feststellung von Veränderungen an ihnen beschränkt. Durch die Weiterentwicklung der $\gamma$-Kamera und ihre Kopplung mit elektronischen Datenverarbeitungsanlagen ist es möglich geworden, Szintigramme in Sekundenabständen aufzunehmen und zu speichern. Mit Hilfe solcher Szintigramm-Sequenzen kann man neben der bildlichen Darstellung eines bestimmten Organs, z. B. der Leber, auch Aussagen über dessen Funktion machen. Man spricht dann von »Funktionsszintigraphie«.

Für Leberfunktionsuntersuchungen verwendete man zunächst mit J-131 markierte lebergängige Substanzen wie z. B. $^{131}$J-Bengalrosa oder $^{131}$J-Bromsulfan. Wegen der relativ hohen Strahlenbelastung und wegen der bei diesen Verbindungen vergleichsweise langsamen Leberpassage, die aus untersuchungstechnischen Gründen unvorteilhaft ist, wurde nach Wegen gesucht, für diese Untersuchungen ein mit Technetium-99m markierbares Diagnostikum mit schnellerer Leberpassage zu finden.

In der Literatur wurden hauptsächlich drei verschiedene Substanzen bzw. Substanzklassen für solche Untersuchungen vorgeschlagen:

1. $^{99m}$Tc-Penicillamin (z. B. G. T. Krishnamurathy, M. Tubis, J. S. Endow et al.: J. Nucl. Med. 13, 447 (1972))
2. $^{99m}$Tc-Pyridoxalaminosäuren, insbesondere $^{99m}$Tc-Pyridoxalglutamat (z. B. R. J. Baker, J. C. Bellen, P. M. Ronai: J. Nucl. Med. 16, 720 (1975))
3. $^{99m}$Tc-Acetanilido-iminodiacetate (z. B. E. Harvey, M. Loberg, M. Cooper: J. Nucl. Med. 16, 533 (1975))

Bei den $^{99m}$Tc-Acetanilido-iminodiacetaten kamen zunächst die beiden Derivate (2,6-Dimethylacetanilido)-iminodiacetat (HIDA) und (2,6-Diäthylacetanilido)-iminodiacetat (EHIDA) zur Anwendung.

In der DE-OS 2 723 605 sind die Verbindungen der Formel

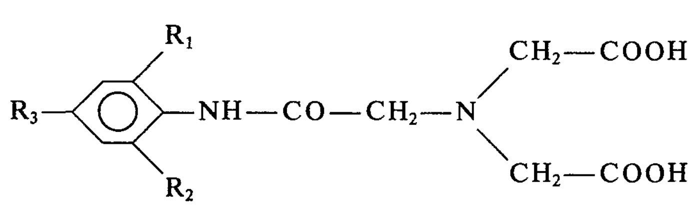

beschrieben, »in welcher mindestens zwei der Symbole $R_1$, $R_2$ und $R_3$ niedere Alkylgruppen mit 1 bis 4 Kohlenstoffatomen bedeuten und die drei Symbole insgesamt mindestens drei Kohlenstoffatome darstellen«. Dabei ist speziell auf die Verbindungen (2,6-Diäthylacetanilido)-iminodiacetat,

(2,6-Diisopropylacetanilido)-iminodiacetat und (2,4,6-Trimethylacetanilido)-iminodiacetat abgehoben.

Alle bisher beschriebenen Derivate des Acetanilido-iminodiacetats werden zu einem bestimmten Anteil über die Niere ausgeschieden, so daß bei der Anwendung auch die Niere dargestellt wird.

Aufgabe der vorliegenden Erfindung ist die Herstellung eines Leberfunktionsdiagnostikums mit möglichst geringer Nierenausscheidung, weil bei Patienten mit eingeschränkter Leberfunktion nur dann noch eine ausreichende Aufnahem des Diagnostikums in die Leber erwartet werden kann, wenn die kompensatorische Ausscheidung über die Nieren weitgehend zurückgedrängt werden kann.

Gegenstand der Erfindung ist ein Verfahren zur Herstellung eines Leberfunktionsdiagnostikums mit extrem geringer Nierenausscheidung, dadurch gekennzeichnet, daß man (2,3,4,5,6-Pentafluoracetanilido)-iminodiacetat (PFIDA) oder (4-n-Pentylacetanilido)-iminodiacetat (PIDA) in wäßriger Lösung mit einem Zinn(II)-Salz im Molverhältnis zwischen 10 : 1 und 200 : 1 mischt, die Lösung auf einen pH-Wert zwischen 4 und 9, vorzugsweise zwischen 5,5 und 6,5 einstellt und anschließend je nach Verwendungszweck 0,1 — 100 mCi Tc-99m-Pertechnetat in physiologischer Kochsalzlösung zugibt. Die Konzentrationen von PFIDA bzw. PIDA liegen zweckmäßig zwischen 0,1 und 200 mg/ml, vorzugsweise zwischen 10 und 50 mg/ml.

Die Konzentration an Zinn(II)-Salz liegt zweckmäßig zwischen 0,01 und 5 mg/ml, vorzugsweise zwischen 0,1 und 0,5 mg/ml Lösung.

Als Zinn(II)-Salz kann $SnF_2$, $SnSO_4$, Sn-tartrat, SnO, Sn-oxalat oder ein anderes Zinn(II)-Salz verwendet werden; bevorzugt ist das Chlorid, insbes. $SnCl_2 \cdot 2\ H_2O$.

Für die Herstellung des Diagnostikums ist es von Vorteil, wenn die (2,3,4,5,6-Pentafluoracetanilido)-iminodiessigsäure unter Zugabe von wäßriger Natronlauge in Wasser gelöst wird. Es wird dabei soviel NaOH zugegeben, wie zum vollständigen Lösen der Verbindung erforderlich ist. Der pH-Wert liegt nach dem Lösen zwischen 7 und 10; er wird dann mit HCl auf ca. 5 eingestellt. Danach wird die entsprechende Menge Sn(II)-Salz (in 0,1 n HCl gelöst) zugegeben und 30 Minuten unter Schutzgasatmosphäre gerührt und der pH auf 6 eingestellt.

Diese Lösung kann innerhalb von 24 Stunden durch Zugabe von Technetium-99m-Pertechnetat zum gebrauchsfertigen Diagnostikum umgesetzt werden. Sie kann aber auch zur Stabilisierung eingefroren oder gefriergetrocknet werden. Das gefriergetrocknete Präparat ist unter Stickstoff-Schutzgas mindestens 8 Monate stabil.

Oft wird die nach dem Verfahren gemäß der Erfindung hergestellte Lösung nicht sogleich mit Technetium-99m-Pertechnetat vereinigt. Es ist dann zweckmäßig, die Lösung zu vereinzeln, indem man sie portionsweise in geeignete Gefäße, z. B. gläserne Rollrandflaschen abfüllt, z. B. in Portionen von 1 oder 2 Millilitern. In diesen Gefäßen wird die Lösung eingefroren oder gefriergetrocknet. Man erhält so in einem Gefäß eine Markierungseinheit. Diese enthält 1 — 200 mg, vorzugsweise 10 — 50 mg PFIDA bzw. PIDA, und daneben 0,01 — 5 mg, vorzugsweise 0,1 — 0,5 mg Zinn(II)-Salz. Zum Gebrauch wird die Markierungseinheit mit 0,1 — 100 mCi, vorzugsweise 1 — 10 mCi in zweckmäßig 1 — 10 ml Pertechnetatlösung pro Patient versetzt.

Im Regelfall erhält ein Patient eine fünftel bis eine ganze markierte Einheit (d. h. fertiges Diagnostikum) intravenös injiziert.

Gegenstand der Erfindung ist somit auch ein Diagnostikum zur Darstellung des hepato-biliären Systems, dadurch gekennzeichnet, daß es mit Technetium-99m markiertes (2,3,4,5,6-Pentafluoracetanilido)-iminodiacetat bzw. (4-n-Pentylacetanilido)-iminodiacetat in physiologischer Kochsalzlösung enthält.

Das neue Diagnostikum zeichnet sich gegenüber den bisher bekannten Verbindungen durch eine wesentlich geringere Anreicherung von Aktivität in den Nieren und eine geringere Ausscheidung in die Harnblase aus. Dieser Vorteil geht aus den im folgenden beschriebenen Tierexperimenten hervor:

1. Einem Kaninchen werden 0,5 mCi des jeweiligen markierten Diagnostikums in die Ohrvene injiziert und die Verteilung der Aktivität mit Hilfe einer $\gamma$-Kamera, die an eine Datenverarbeitungsanlage angeschlossen ist, in ihrem zeitlichen Verlauf verfolgt.

Während bei den bisher bekannten Präparaten immer auch die Nieren mit dargestellt werden, sind sie bei den neuen Diagnostika PFIDA und PIDA praktisch nicht zu lokalisieren.

2. Gruppen von jeweils 12 Ratten, denen zuvor zur Bestimmung der Blasenaktivität die abführenden Harnwege abgebunden wurden, werden je 30 $\mu$Ci des jeweiligen Diagnostikums in die Oberschenkelvene injiziert. Dann werden je 3 Tiere nach 5, 10, 20 und 30 Minuten geötet und die Organverteilung der Radioaktivität bestimmt. In der folgenden Tabelle sind die Ergebnisse zusammengefaßt:

| Substitution am Aromaten | Zeit | Leber (%) | Darm (%) | Nieren (%) | Blase (%) |
|---|---|---|---|---|---|
| 2,6-Dimethyl- (HIDA) | 5′ | 21,7 | 43,2 | 7,4 | 0,9 |
| | 10′ | 9,6 | 65,9 | 7,4 | 1,7 |
| | 20′ | 2,2 | 80,8 | 5,8 | 2,3 |
| | 30′ | 1,0 | 79,7 | 4,9 | 2,8 |
| 2,6-Diäthyl- (EHIDA) | 5′ | 6,9 | 71,2 | 4,3 | 0,4 |
| | 10′ | 3,4 | 79,9 | 4,2 | 1,3 |
| | 20′ | 1,6 | 81,7 | 3,6 | 1,6 |
| | 30′ | 1,5 | 81,8 | 3,4 | 1,8 |
| 2,3,4,5,6-Pentafluor- (PFIDA) | 5′ | 28,3 | 35,6 | 2,4 | 0,8 |
| | 10′ | 14,1 | 64,8 | 2,5 | 1,1 |
| | 20′ | 5,8 | 78,3 | 1,7 | 1,7 |
| | 30′ | 3,8 | 81,8 | 1,6 | 1,8 |
| 4-n-Pentyl-IDA (PIDA) | 5′ | 65,6 | 24,6 | 1,0 | 0,04 |
| | 10′ | 46,5 | 46,9 | 1,0 | 0,05 |
| | 20′ | 22,2 | 73,4 | 1,1 | 0,09 |
| | 30′ | 12,5 | 85,4 | 1,0 | 0,13 |

Vor allem bei der Verbindung PIDA wird kaum eine Aktivitätsausscheidung in die Harnblase beobachtet.

## Beispiel 1

### Herstellung von (2,3,4,5,6-Pentafluoracetanilido)-iminodiacetat

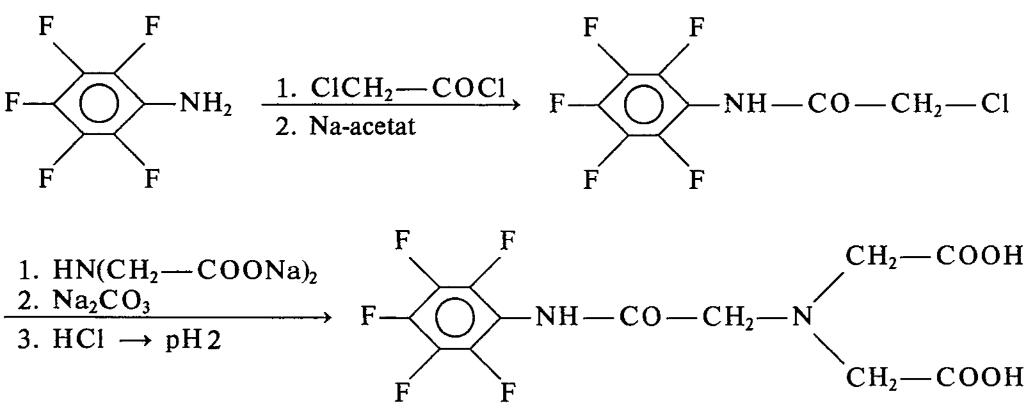

a) 18,31 g (0,1 mol) 2,3,4,5,6-Pentafluoranilin werden in 80 ml Eisessig gelöst. Die Lösung wird auf 10°C gekühlt, mit 12,4 g (0,11 mol) Chloressigsäurechlorid versetzt und kräftig durchmischt. Dann werden unter starkem Rühren 100 ml halb gesättigte wäßrige Natriumacetat-Lösung und 100 ml Wasser zugegeben und 30 Minuten gerührt. Das ausgefallene Chloressigsäure-(2,3,4,5,6-Pentafluoranilid) wird abgesaugt und aus Methanol umkristallisiert.

Schmelzpunkt:
139 – 141°C
Elementaranalyse:

| | | | | |
|---|---|---|---|---|
| ber. | C 37,02% | H 1,16% | N 5,40% | Cl 13,66% |
| gef. | C 37,0% | H 1,3% | N 5,6% | Cl 13,7% |

NMR (in DMSO-$d_6$, interner Standard: TMS):
$-CH_2-$: $\delta$ = 4,37 ppm (s)
$-NH-$: $\delta$ = 10,42 ppm (s)

b) 4,0 g (22 mmol) Iminodiessigsäure-Dinatriumsalz, 5,7 g (20 mmol) $Na_2SO_3 \cdot 10\,H_2O$ und 5,2 g (20 mmol) Chloressigsäure-(2,3,4,5,6-Pentafluoranilid) werden in 60 ml Wasser und 30 ml Äthanol aufgenommen und 24 Stunden am Rückfluß gekocht. Nach dem Abkühlen wird das Lösungsmittel abgezogen und der Rückstand in ca. 150 ml Wasser gelöst. Diese wäßrige Lösung wird 4 × mit ca. 50 ml Äther gewaschen und anschließend mit HCl langsam auf pH 2 gebracht. Das angefallene Produkt wird abgesaugt, wieder in Wasser gelöst, wobei soviel NaOH zugegeben wird, wie zum vollständigen Lösen der Substanz erforderlich ist. Nach Filtrieren wird (2,3,4,5,6-Pentafluoracetanilido)-iminodiessigsäure durch Zugabe von HCl bis pH 2 wieder ausgefällt, abgesaugt und getrocknet.

Schmelzpunkt:
181 – 184°C
Elementaranalyse:

| | | | |
|---|---|---|---|
| ber. | C 40,46% | H 2,55% | N 7,86% |
| gef. | C 40,7% | H 2,8% | N 7,8% |

NMR (in DMSO-$d_6$, int. Standard: TMS):
$>N-CH_2-COOH$: $\delta$ = 3,56 ppm (s)
$-CO-CH_2-N<$: $\delta$ = 3,61 ppm (s)
$-NH-$: / $-COOH$: } $\delta \approx$ 11,5 ppm (s)*)

*) Durch die Fluorsubstitution am Aromaten ist das NH-Proton so stark acidifiziert, daß es mit dem im Lösungsmittel vorhandenen Wasser und den Säureprotonen schnell austauscht, weshalb nur ein Signal mit einer gemittelten chemischen Verschiebung auftritt.

## Beispiel 2

Die Synthese von (4-n-Pentylacetanilido)-iminodiessigsäure erfolgt analog Beispiel 1 aus 4-n-Pentylanilin über Chloressigsäure-(4-n-Pentylanilid). Man erhält folgende physikalischen Parameter:

Schmelzpunkt:
192 – 194°C
Elementaranalyse:

| | | | |
|---|---|---|---|
| ber. | C 60,70% | H 7,19% | N 8,33% |
| gef. | C 61,0% | H 7,0% | N 8,2% |

NMR (in DMSO-$d_6$; interner Standard: TMS):

| | | |
|---|---|---|
| $>N-CH_2-COOH$: | $\delta$ = 3,55 ppm (s) | |
| $-CO-CH_2-N$: | $\delta$ = 3,47 ppm (s) | |
| $-NH<$: | $\delta$ = 10,13 ppm (s) | |
| $-COOH$: | $\delta$ = 12,5 ppm (s, breit) | |
| Aromaten ($A_2B_2$-System): | $\delta_A$ = 7,11 ppm | $J_{AB}$ = 8 Hz |
| | $\delta_B$ = 7,50 ppm | |
| $-CH_2-(\alpha)$: | $\delta$ = 2,54 ppm (t); J = 7 Hz | |
| $-(CH_2)_3-(\beta-\delta)$: | $\delta$ = 1,0 – 1,7 ppm (m) | |
| $-CH_3\,(\varepsilon)$: | $\delta$ = 0,86 ppm (t); J = 6 Hz | |

Beispiel 3

7 g (2,3,4,5,6-Pentafluoracetanilido)-iminodiessigsäure werden in 100 ml 1 N Natronlauge gelöst und mit 70 mg $SnCl_2 \cdot 2 H_2O$ (gelöst in 7 ml 0,1 N Salzsäure) versetzt. Nach 5 Minuten Rühren wird der pH-Wert mit Salzsäure auf 6 eingestellt, auf 233 ml aufgefüllt, in 1 ml-Portionen in Rollrandflaschen abgefüllt und mit flüssigem Stickstoff eingefroren. Anschließend wird das Präparat gefriergetrocknet, die Flaschen werden mit Stickstoff gefüllt und verschlossen.

Nach einer Lagerzeit von 60 Tagen werden in die verschlossenen Flaschen 0,3 mCi Tc-99m-Pertechnetat in 10 ml physiologischer Kochsalzlösung injiziert. Nach ca. 30 Minuten Reaktionszeit werden je 0,5 ml des so gewonnenen Diagnostikums Ratten intravenös appliziert (typische Versuchsergebnisse können der Tabelle auf S. 6 entnommen werden).

Beispiel 4

3 g (4-n-Pentylacetanilido)-iminodiessigsäure werden unter Zusatz von soviel 1 n NaOH, wie zum vollständigen Lösen der Substanz erforderlich ist, in ca. 60 ml Wasser gelöst und mit 20 mg $SnF_2$ (gelöst in 5 ml 0,1 N Salzsäure) versetzt. Nach ca. 10 Minuten Rühren wird auf pH 6 eingestellt und auf 100 ml aufgefüllt. Die Lösung wird in 1-ml-Portionen in Rollrandflaschen abgefüllt und mit flüssigem Stickstoff eingefroren. Das Präparat wird anschließend gefriergetrocknet, die Flaschen werden mit Stickstoff gefüllt und verschlossen.

Nach einer Lagerzeit zwischen 1 Tag und 15 Monaten wird die Markierungseinheit entsprechend Beispiel 3 injektionsfertig gemacht und Ratten appliziert (typische Versuchsergebnisse können der Tabelle auf S. 6 entnommen werden).

**Patentansprüche für die Vertragsstaaten: BE, CH, LI, DE, FR, GB, IT, LU, NL, SE**

1. Verfahren zur Herstellung eines Leberfunktionsdiagnostikums, dadurch gekennzeichnet, daß man (2,3,4,5,6-Pentafluoracetanilido)-iminodiacetat (PFIDA) und/oder (4-n-Pentylacetanilido)-iminodiacetat (PIDA) in wäßriger Lösung mit einem Zinn(II)-Salz im Molverhältnis zwischen 10 : 1 und 200 : 1 mischt, die Lösung auf einen pH-Wert zwischen 4 und 9, vorzugsweise zwischen 5,5 und 6,5, einstellt und anschließend je nach Verwendungszweck 0,1 – 100 mCi Technetium-99m-Pertechnetat in physiologischer Kochsalzlösung zugibt.

2. Verfahren nach Anspruch 1, dadurch gekennzeichnet, daß die Konzentration von PFIDA bzw. PIDA zweckmäßig zwischen 0,1 und 200 mg/ml, vorzugsweise zwischen 10 und 50 mg/ml, liegt.

3. Verfahren nach Anspruch 1, dadurch gekennzeichnet, daß die wäßrige Lösung von PFIDA bzw. PIDA und Zinn(II)-Salz vor der Zugabe der Pertechnetatlösung in kleinen Portionen (Markierungseinheiten) gefriergetrocknet wird.

4. Verfahren nach Anspruch 3, dadurch gekennzeichnet, daß eine Portion (Markierungseinheit) 1 bis 200 mg, vorzugsweise 10 bis 50 mg, PFIDA bzw. PIDA sowie 0,01 bis 5 mg, vorzugsweise 0,1 bis 0,5 mg, Zinn(II)-Salz enthält.

5. Verfahren nach den Ansprüchen 1 – 4, dadurch gekennzeichnet, daß als Zinn(II)-Salz $SnCl_2 \cdot 2 H_2O$, $SnF_2$, $SnSO_4$, Sn-tartrat, SnO, Sn-oxalat, Sn-acetat oder ein anderes Zinn(II)-Salz verwendet wird.

6. Diagnostikum zur Darstellung des hepato-biliären Systems, dadurch gekennzeichnet, daß es mit Technetium-99m markiertes (2,3,4,5,6-Pentafluoracetanilid)-iminodiacetat in physiologischer Kochsalzlösung enthält.

7. Diagnostikum zur Darstellung des hepato-biliären Systems, dadurch gekennzeichnet, daß es mit Technetium-99m markiertes (4-n-Pentylacetanilido)-iminodiacetat in physiologischer Kochsalzlösung enthält.

**Patentansprüche für den Vertragsstaat: AT**

1. Verfahren zur Herstellung eines Leberfunktionsdiagnostikums, dadurch gekennzeichnet, daß man (2,3,4,5,6-Pentafluoracetanilido)-iminodiacetat (PFIDA) und/oder (4-n-Pentylacetanilido)-iminodiacetat (PIDA) in wäßriger Lösung mit einem Zinn(II)-Salz im Molverhältnis zwischen 10 : 1 und 200 : 1 mischt, die Lösung auf einen pH-Wert zwischen 4 und 9, vorzugsweise zwischen 5,5 und 6,5, einstellt und anschließend je nach Verwendungszweck 0,1 – 100 mCi Technetium-99m-Pertechnetat in physiologischer Kochsalzlösung zugibt.

2. Verfahren nach Anspruch 1, dadurch gekennzeichnet, daß die Konzentration von PFIDA bzw. PIDA zweckmäßig zwischen 0,1 und 200 mg/ml, vorzugsweise zwischen 10 und 50 mg/ml, liegt.

3. Verfahren nach Anspruch 1, dadurch gekennzeichnet, daß die wäßrige Lösung von PFIDA bzw. PIDA und Zinn(II)-Salz vor der Zugabe der Pertechnetatlösung in kleinen Portionen (Markierungseinheiten) gefriergetrocknet wird.

4. Verfahren nach Anspruch 3, dadurch gekennzeichnet, daß eine Portion (Markierungseinheit) 1 bis 200 mg, vorzugsweise 10 bis 50 mg, PFIDA bzw. PIDA sowie 0,01 bis 5 mg, vorzugsweise 0,1 bis 0,5 mg, Zinn(II)-Salz enthält.

5. Verfahren nach den Ansprüchen 1—4, dadurch gekennzeichnet, daß als Zinn(II)-Salz $SnCl_2 \cdot 2\,H_2O$, $SnF_2$, $SnSO_4$, Sn-tartrat, SnO, Sn-oxalat, Sn-acetat oder ein anderes Zinn(II)-Salz verwendet wird.

**Claims for the Contracting States: BE, CH, LI, DE, FR, GB, IT, LU, NL, SE**

1. A process for the manufacture of a diagnostic agent for liver function studies, which comprises mixing (2,3,4,5,6-pentafluoroacetanilido)-iminodiacetate (PFIDA) and/or (4-n-pentylacetanilido)-iminodiacetate (PIDA) in aqueous solution with a tin(II) salt in a molar ratio of from 10 : 1 to 200 : 1, adjusting the pH of the resulting solution to a value between 4 and 9, preferably between 5.5 and 6.5, and subsequently adding from 0.1 to 100 mCi of Tc-99m pertechnetate in physiological saline solution, depending on the intended purpose.

2. A process as claimed in claim 1, wherein the concentration of PFIDA or PIDA respectively, is suitably in the range of from 0.1 to 200 mg/ml, preferably of from 10 to 50 mg/ml.

3. A process as claimed in claim 1, which comprises lyophilizing the aqueous solution of PFIDA or PIDA respectively, and of tin(II) salt, in small portions (labelling units), prior to adding the pertechnetate solution.

4. A process as claimed in claim 3, wherein one portion (labelling unit) contains of from 1 to 200 mg, preferably from 10 to 50 mg, of PFIDA or PIDA respectively, and of from 0.01 to 5 mg, preferably from 0.1 to 0.5 mg of tin(II) salt.

5. A process as claimed in claims 1 to 4, which comprises using as tin(II) salt $SnCl_2 \cdot 2\,H_2O$, $SnF_2$, $SnSO_4$, Sn-tartrate, SnO, Sn-oxalate, Sn-acetate or another tin(II) salt.

6. A diagnostic agent for visualizing the hepatobiliary system, which comprises (2, 3, 4, 5, 6-pentafluoroacetanilido)-iminodiacetate labelled with technetium-99m, in physiological saline solution.

7. A diagnostic agent for visualizing the hepatobiliary system, which comprises (4-n-pentylacetanilido)-iminodiacetate labelled with technetium-99m, in physiological saline solution.

**Claims for the Contracting State: AT**

1. A process for the manufacture of a diagnostic agent for liver function studies, which comprises mixing (2,3,4,5,6-pentafluoroacetanilido)-iminodiacetate (PFIDA) and/or (4-n-pentylacetanilido)-iminodiacetate (PIDA) in aqueous solution with a tin(II) salt in a molar ratio of from 10 : 1 to 200 : 1, adjusting the pH of the resulting solution to a value between 4 and 9, preferably between 5.5 and 6.5, and subsequently adding from 0.1 to 100 mCi of Tc-99m pertechnetate in physiological saline solution, depending on the intended purpose.

2. A process as claimed in claim 1, wherein the concentration of PFIDA or PIDA respectively, is suitably in the range of from 0.1 to 200 mg/ml, preferably of from 10 to 50 mg/ml.

3. A process as claimed in claim 1, which comprises lyophilizing the aqueous solution of PFIDA or PIDA respectively, and ot tin(II) salt, in small portions (labelling units), prior to adding the pertechnetate solution.

4. A process as claimed in claim 3, wherein one portion (labelling unit) contains of from 1 to 200 mg, preferably from 10 to 50 mg, of PFIDA or PIDA respectively, and of from 0.01 to 5 mg, preferably from 0.1 to 0.5 mg of tin(II) salt.

5. A process as claimed in claims 1 to 4, which comprises using as tin(II) salt $SnCl_2 \cdot 2\,H_2O$, $SnF_2$, $SnSO_4$, Sn-tartrate, SnO, Sn-oxalate, Sn-acetate or another tin(II) salt.

**Revendications pour les Etats contractants: BE, CH, LI, DE, FR, GB, IT, LU, NL, SE**

1. Procédé pour la préparation d'un agent de diagnostic de la fonction du foie, caractérisé en ce qu'on mélange du (2,3,4,5,6-pentafluoroacétanilido)-iminodiacétate (PFIDA) et/ou du (4-n-pentylacétanilido)-iminodiacétate (PIDA) en solution aqueuse avec un sel d'étain(II) dans un rapport molaire compris entre 10 : 1 et 200 : 1, on ajuste le pH de la solution à une valeur comprise entre 4 et 9, de préférence entre 5,5 et 6,5 et selon l'application envisagée on ajoute de 0,1 à 100 mCi de pertechnétate marqué au technétium-99m dans une solution saline physiologique.

2. Procédé selon la revendication 1, caractérisé en ce que la concentration du PFIDA ou du PIDA est compris avantageusement entre 0,1 et 200 mg/ml, de préférence entre 10 et 50 mg/ml.

3. Procédé selon la revendication 1, caractérisé en ce que la solution aqueuse de PFIDA ou de

PIDA et de sel d'étain(II) est lyophilisée en petites portions (unités de marquage) avant l'addition de la solution de pertechnétate.

4. Procédé selon la revendication 3, caractérisé en ce qu'une portion (unité de marquage) contient de 1 à 200 mg, de préférence de 10 à 50 mg de PFIDA ou de PIDA ainsi que de 0,01 à 5 mg, de préférence de 0,1 à 0,5 mg de sel d'étain(II).

5. Procédé selon l'une quelconque des revendications 1 à 4, caractérisé en ce que comme sel d'étain (II), on utilise $SnCl_2 \cdot 2\,H_2O$, $SnF_2$, $SnSO_4$, le tartrate d'étain, SnO, l'oxalate d'étain, l'acétate d'étain ou un autre sel d'étain(II).

6. Agent de diagnostic pour la visualisation du système hépato-biliaire, caractérisé en ce qu'il contient du (2,3,4,5,6-pentafluoroacétanilido)-iminodiacétate marqué avec du technétium-99m en solution saline physiologique.

7. Agent de diagnostic pour la visualisation du système hépato-biliaire, caractérisé en ce qu'il contient du (4-n-pentylacétanilido)-iminodiacétate marqué avec du technétium-99m en solution saline physiologique.

**Revendications pour l'Etat contractant: AT**

1. Procédé pour la préparation d'un agent de diagnostic de la fonction du foie, caractérisé en ce qu'on mélange du (2,3,4,5,6-pentafluoroacétanilido)-iminodiacétate (PFIDA) et/ou du (4-n-pentylacétanilido)-iminodiacétate (PIDA) en solution aqueuse avec un sel d'étain(II) dans un rapport molaire compris entre 10 : 1 et 200 : 1, on ajuste le pH de la solution à une valeur comprise entre 4 et 9 de préférence entre 5,5 et 6,5, puis selon l'application envisagée, on ajoute de 0,1 à 100 mCi de pertechnétate marqué au technétium-99m en solution saline physiologique.

2. Procédé selon la revendication 1, caractérisé en ce que la concentration du PFIDA ou du PIDA est comprise avantageusement entre 0,1 et 200 mg/ml, de préférence entre 10 et 50 mg/ml.

3. Procédé selon la revendication 1, caractérisé en ce que la solution aqueuse de PFIDA ou de PIDA et de sel d'étain(II) est lyophilisée en petites portions (unités de marquage) avant d'ajouter la solution du pertechnétate.

4. Procédé selon la revendication 3, caractérisé en ce qu'une portion (unité de marquage) contient de 1 à 200 mg, de préférence de 10 à 50 mg, de PFIDA ou de PIDA et de 0,01 à 5 mg de préférence de 0,1 à 0,5 mg de sel d'étain(II).

5. Procédé selon l'une quelconque des revendications 1 à 4, caractérisé en ce que comme sel d'étain(II), on utilise $SnCl_2 \cdot 2\,H_2O$, $SnF_2$, $SnSO_4$, le tartrate d'étain, SnO, l'oxalate d'étain, l'acétate d'étain ou un autre sel d'étain(II).